# EUROPÄISCHE PATENTSCHRIFT

(11) **EP 1 988 202 B1**
(45) Veröffentlichungstag und Bekanntmachung des Hinweises auf die Patenterteilung: **05.12.2012**
(21) Anmeldenummer: 08007752.2
(22) Anmeldetag: 22.04.2008
(51) Int. Cl.: D04H 3/14, D04H 3/16, A61F 13/20

(54) **Vliesstoffüberzug mit niedrigem Reibungskoeffizienten für die Frauenhygiene, insbesondere für Tampons, oder für medizinische Zwecke, sowie damit versehene Artikel**
Non-woven fabric cover with low friction coefficients for feminine hygiene, in particular for tampons or for medical purposes, and articles equipped with same
Revêtement en tissu non tissé avec un coefficient de friction bas pour l'hygiène féminine, en particulier pour des tampons, ou à des fins médicales, et article en étant équipé

(30) Priorität: 04.05.2007 DE 102007021374
(43) Veröffentlichungstag der Anmeldung: 05.11.2008
(73) Patentinhaber: Carl Freudenberg KG, 69469 Weinheim (DE)
(72) Erfinder: Kamin-Schulz, Elisabeth, 66862 Kindsbach (DE); Knehr, Eric, 67688 Rodenbach (DE); Hess, Michael, 67705 Trippstadt (DE); Rutsch, Peter, 69518 Absteinach (DE)

(56) Entgegenhaltungen:
- EP-A- 1 498 093
- EP-A- 1 505 187
- WO-A-01/71080
- WO-A-99/27878
- WO-A-99/32061
- WO-A-2005/090659
- US-A- 5 817 077
- US-A1- 2002 094 741

## Beschreibung

### Technisches Gebiet

Die Erfindung betrifft mit Vliesstoffüberzugen versehene Tampons.

### Stand der Technik

Vliesstoffüberzüge finden auf vielen Gebieten Einsatz, beispielsweise in der Frauenhygiene oder bei medizinischen Artikeln, bei welchen diesen sowohl funktionale als auch ästhetische Bedeutung zukommt. Funktional können durch den Überzug Eigenschaften der Oberfläche positiv beeinflusst werden, wie beispielsweise deren Reibungskoeffizient, deren Abriebfestigkeit oder kann auch die mechanische Stabilität des Artikels selbst verbessert werden, falls dieser beispielsweise durch Aufnahme von Körperflüssigkeiten an Festigkeit verliert.

Aus der US 3,683,912 A ist ein Tampon bekannt, welcher eine äußere flüssigkeitsdurchlässige Lage aus Polypropylen-Fasem aufweist. Diese Umhüllung ist vorzugsweise als punktweise geprägter Vliesstoff ausgebildet.

Die EP 1 035 819 B1 zeigt einen Tampon für die Frauenhygiene sowie ein Herstellungsverfahren für diesen, bei welchem ein Vliesstoff-Überzug, der zumindest teilweise thermoplastische, heißsiegelbare Fasern umfasst, vorgesehen ist. Dieser Vliesstoff-Überzug wird dabei aus Stapelfasern hergestellt. Um den Reibungskoeffizienten dieses Vliesstoffüberzugs zu vermindern, wird vorgeschlagen, diesen durch Kalandrieren mit glatten Kalanderwalzen zu glätten. Diese Glättung soll zwar eine weiter verringerte Haftreibung zur Folge haben, allerdings verringern sich auch die Porengrößen, da die Fasern beim Kalandrieren flachgedrückt und miteinander verschmolzen werden.

Auch aus der US 4,056,103 A ist weiterhin ein Tampon mit einer Vlieshülle aus Stapelfasern bekannt.

Ein generelles Problem bei den aus dem Stand der Technik bekannten Vliesstoff-Überzügen besteht insbesondere auch darin, dass bei den üblicherweise verwendeten Stapelfasern die Faserenden freilegen. Diese erhöhen die Rauhigkeit und erniedrigen den Tragekomfort. Zudem kann die definierte Schnittlänge in Zusammenhang mit freiliegenden Faserenden zu erhöhtem Faserverlust führen.

### Darstellung der Erfindung

Ausgehend von dem oben beschriebenen Stand der Technik liegt der vorliegenden Erfindung daher die Aufgabe zugrunde, einen Vliesstoff-Überzug für Tampons bereitzustellen, welcher eine geringe Reibung mit hoher Dehnfähigkeit vereint und die Nachteile durch offenliegende Faserenden vermeidet. Diese Aufgabe wird durch den Gegenstand des Anspruchs 1 gelöst. Vorteilhafte Ausgestaltungen und Weiterbildungen der Erfindung sind in den abhängigen Ansprüchen angegeben.

Die Erfindung sieht demgemäß Tampons mit einem Vliesstoffüberzug, insbesondere für die Frauenhygiene oder für medizinische Zwecke vor. Der Vliesstoffüberzug umfasst wenigstens eine Lage mit Mehrkomponentenfilamenten, die wenigstens eine Komponente aus einem thermoplastischen heißsiegelbaren Material enthalten, wobei die Mehrkomponentenfilamente Endlosfilamente sind, und wobei die Endlosfilamente in ersten Bereichen zusammengepresst und unter Aufschmelzen des thermoplastischen heißsiegelbaren Materials durch thermische Verbindung festgelegt sind und in zweiten Bereichen zwischen den ersten heißgesiegelten, beziehungsweise durch thermische Verbindung festgelegten Bereichen unverbunden vorliegen.

Die Filamente können beispielsweise eine Kern-Mantel-Geometrie aufweisen. In diesem Fall wird zur Erzielung einer geringen Reibung vorzugsweise das Material mit dem geringeren Reibungskoeffizienten als Mantelmaterial eingesetzt.

Vorzugsweise bilden aber zumindest zwei der Komponenten, bei Bikomponenten-Filamenten dementsprechend alle Komponenten Bereiche der Filamentoberfläche.

Erfindungsgemäß werden also Endlosfilamente anstelle von Stapelfasern verwendet. Als Stapelfasern werden dabei Fasern verstanden, die Längen bis in den Dezimeterbereich hinein aufweisen. Dadurch, dass eine Festlegung der Filamente nur bereichsweise erfolgt, ist eine flächige Glättung, wie sie die EP 1 035 819 B1 vorschlägt, nicht vorhanden. Es wird erfindungsgemäß dennoch ein Vliesstoff mit einer Oberfläche bereitgestellt, die einen sanften Eindruck aufweist. Durch die Verwendung von Endlosfilamenten, und damit auch unter Vermeidung loser Faserenden kann überraschenderweise auch auf einen Faserquerschnitt verzichtet werden, bei dem die gesamte Oberfläche der Filamente von einem Polymer mit niedrigem Reibkoeffizienten gebildet wird. Ein günstiger Einfluss ist sogar dann, beziehungsweise im Besonderen vorzufinden, wenn besagtes reibungsarmes Polymer nur segmentweise an der Oberfläche der Filamente vorhanden ist. In diesem Fall kann bei Erhalt eines niedrigen Reibungskoeffizienten auf eine mechanische und/oder thermische Glättung verzichtet werden. Es ist daher vorgesehen, dass die verschiedenen Komponenten der Filamente, beziehungsweise bei mehr als zwei Komponenten wenigstens zwei der Komponenten auch jeweils Teile der Mantelfläche der Filamente bilden, Dies stellt einen erheblichen Vorteil gegenüber bekannten Vliesstoffüberzügen dar, bei welchen Kern-Mantel-Fasern verwendet werden, bei denen der gesamte Mantel zur Reduzierung der Reibung aus einem reibungsarmen Polymer besteht.

Durch die Endlosfilamente werden gleichzeitig hervorstehende Faserenden vermieden, so dass dennoch eine glatte Oberfläche erhalten wird. Demgemäß wird der Vliesstoff bevorzugt überwiegend, besonders bevorzugt ausschließlich aus Endlosfilamenten hergestellt. Es wird aber nicht ausgeschlossen, dass auch ein geringer Anteil von Stapelfasern vorhanden sein kann, ohne dass die Vorteile der Erfindung im Wesentlichen verloren gehen, sofern der Anteil freiliegender Faserenden hinreichend klein ist.

Die Mehrkomponentenfüamente verhindern weiterhin, dass beim lokalen, beziehungsweise bereichsweisen Heißsiegeln an den Rändern der ersten Bereiche die Filamente nicht oder zumindest seltener derart zusammengequetscht und in ihrem Querschnitt derart verringert werden, dass es zu einer Trennung des Filaments und damit einhergehend zur Entstehung freiliegender Enden kommt. Dies bewirkt eine gegenüber den gemäß der US 3,683,912 A vorgeschlagenen Polypropylen-Fasem verbesserte Festigkeit, Glätte und auch Elastizität.

Durch den Einsatz von Endlos-Mehrkomponentenfilamenten wird außerdem eine gegenüber Stapelfaser-Vliesen erhöhte Höchstzugkraft erreicht.

Unter einem Mehrkomponenten-Filament im Sinne der Erfindung ist dabei insbesondere ein Filament zu verstehen, bei welchem die verschiedenen Komponenten unterschiedliche Bereiche des Filament-Querschnitts und dessen Oberfläche einnehmen. Die Komponenten können sich insbesondere in ihren Schmelz- oder Erweichungstemperaturen unterscheiden. In bevorzugter Ausgestaltung der Erfindung werden Bikomponentenfilamente mit einer thermoplastischen niedrigschmelzenden und einer hochschmelzenden Komponente verwendet. Die unterschiedlichen Schmelzpunkte ermöglichen es, dass beim lokalen Heißsiegeln die niedrigschmelzenden Bereiche der Filamente miteinander verschmelzen, während die höherschmelzenden Teile ihre Struktur im wesentlichen beibehalten, so dass diese Teile durchgehende Stränge in den festgelegten, beziehungsweise verschmolzenen ersten Bereichen ergeben. Damit wird unter anderem erreicht, dass sich Filamente an den Rändern der ersten Bereiche durch Aufschmelzen nicht abtrennen und offene Enden bilden.

Weiterhin ist es bevorzugt, wenn die hochschmelzende Komponente aus der Gruppe der Polyesterderivate und Mischungen derselben ausgewählt ist. Besonders bevorzugt kommen dabei Polyesterderivate aus der Gruppe Polyethylenterephthalat (PET), Polytrimethylenterephthalat (PTT), Polybutylenterephthalat (PBT) und Polymilchsäure (PLA) zum Einsatz. Für die niedrigschmelzende Komponente sind Polymere aus der Gruppe der Polyolefine, insbesondere Polyethylen und Polypropylen geeignet. Eine Kombination mit einem Polyester-Derivat verbessert gegenüber reinen Polyethylen- oder Polypropylen-Fasem das Benetzungsverhalten des Vlieses und führt damit zu einer besseren Weiterleitung der Flüssigkeit in das flüssigkeitsabsorbierende Innenteil des Tampons, welches vom Vliesstoffüberzug umgeben ist.

Dieser Vorteil wird insbesondere mit Bikomponentenfilamenten mit einer so genannten Pie-Geometrie erreicht. Es ist weiterhin aber auch möglich, Multikomponenten-Filamente mit einer Pie-Geometrie einzusetzen. Neben einer Pie-Geometrie ist auch eine so genannte Side-by-side Geometrie möglich, bei welcher die Komponenten im Querschnitt nebeneinander liegende Abschnitte einnehmen.

Bei Bikomponentenfilamenten mit Pie-Geometrie liegen die beiden Komponenten in Form von sich in Längsrichtung entlang des Filaments erstreckenden Strängen vor, wobei die Stränge im Querschnitt des Filaments betrachtet sektorförmig sind und sich entlang des Umfangs Sektoren der beiden Materialien abwechseln.

Bisher werden für reibungsarme Fasern vielfach Kern-Mantel-Fasern eingesetzt, bei denen der Mantel durch reibungsarmes, jedoch teures Polyetylen gebildet wird. Auf diese Weise wird ein Vlies erhalten, welches die Oberflächeneigenschaften von PE aufweist, dessen weitere Eigenschaften aber durch das Material des Kerns modifiziert wird. Man würde daher erwarten, dass eine Filamentgeometrie, bei der sowohl reibungsarmes, als auch reibungsstärkeres Polymer anteilig die Filamentoberfläche bilden, ein Vlies ergibt, welches eine weniger ansprechende Gesamtoberfläche aufweist. Dies ist bei einem erfindungsgemäßen Vlies mit Filamenten, die eine Pie-Geometrie aufweisen, in überraschender Weise gerade nicht der Fall. Obwohl die Oberfläche aus zwei Polymeren gebildet wird, ist sie reibungsarm.

Zudem kommt bei Verwendung von hydrophoben Polyolyfinen als Komponente das im allgemeinen bessere Benetzungsverhalten der anderen Komponente zum Tragen. Allgemein, unabhängig von der Art der Komponenten können sich beim Festlegen durch Aufschmelzen und Pressen die sektorförmigen Stränge der höherschmelzenden Komponente eines Filaments trennen und mit der niedrigerschmelzenden Komponente besser verzahnen. Damit wird auch bei sehr kleinflächiger Festlegung eine reißfestere Struktur erzielt. Vorzugsweise werden dazu Bikomponenten-Filamente mit zumindest 6 Sektoren, also jeweils drei Sektoren eines jeden Materials verwendet. Für die Anwendung besonders günstige Pie-Filamente umfassen Bikomponenten-Filamente aus PET/PE.

Weiterhin bevorzugt sind Bikomponentenfilamente mit Kem-Mantel-Geometrie, bei denen, wie aus dem Stand der Technik bekannt, der Mantel durch ein reibungsarmes Material gebildet wird. Besonders bevorzugt sind Kem-Mantel-Filamente auf Basis PET / PE, wobei PET die Kern- und PE die Mantel-Komponente bildet.

In vorteilhafter Ausgestaltung der Erfindung beträgt der Anteil an PET in den Pie-Filamenten oder Kem-Mantel-Filamenten zwischen 10 und 90 Gewichtsprozent. Bevorzugt werden dabei Anteile von mehr als 40, besonders bevorzugt zumindest 50 Gewichtsprozent, insbesondere im Bereich von 60 bis 90 Gewichtsprozent.

Gemäß einer Weiterbildung der Erfindung kann eine zusätzliche Erniedrigung der Reibwerte auch durch eine Hydrophilierung der Filamente erfolgen.

Die ersten Bereiche, in welchen die Filamente miteinander verbunden sind, sind weiterhin vorzugsweise in der Fläche vollständig von zweiten Bereichen umgeben, um eine allseitige Dehnfähigkeit des Vlieses zu erreichen. Die ersten Bereiche können dazu insbesondere als über die Oberfläche verteilte, voneinander beabstandete Bindungspunkte ausgestaltet sein.

Günstig für die mechanischen Eigenschaften des Vliewtoffüberzugs, sowohl hinsichtlich der Festigkeit, als auch in Bezug auf eine Verminderung der Reibung, sowie für eine homogene Erscheinung der Oberfläche ist weiterhin eine große Dichte von Bindungspunkten auf der Oberfläche. Vorzugsweise beträgt die Flächendichte der Bindungspunkte dabei mehr als 60 Punkte pro Quadratzentimeter. Ebenso ist es für die vorgenannten Eigenschaften günstig, wenn ersten Bereiche, wie insbesondere die Bindungspunkte laterale Abmessungen kleiner als 1 Millimeter oder jeweils Flächen kleiner als 1 Quadratmillimeter aufweisen.

Für eine gute Durchlässigkeit des Vliesstoffes wird weiterhin bevorzugt, wenn die Fläche der ersten Bereiche einen Anteil an der Gesamtoberfläche hat, der 30%, vorzugsweise 25% nicht überschreitet.

Um den Vliesstoffüberzug herzustellen, werden die Mehrkomponenten-Endlosfilamente gesponnen und auf einer Unterlage abgelegt. Das Verfestigen durch Verschmelzen der ersten Bereiche erfolgt dann mittels einer erhitzten Rasterwalze, deren erhabene, erwärmte Strukturen den Flor zusammenpressen und die Filamente dort verschmelzen. Zur Herstellung von. Tampons, wird dann das absorbierende Material mit dem Vliesstoffüberzug zusammengelegt und der Vliesstoffüberzug mit dem absorbierenden Material in der gewünschten Form fixiert.

Erfindungsgemäß eingesatzte Vliesstoffüberzüge zeichnen sich durch eine geringe Reibung und damit einhergehend hohe Glätte, durch eine geringe Adhäsion zur Haut und durch eine sehr gute Weiterverarbeitbarkeit aufgrund der einfachen Verschweißbarkeit mit weiteren Materialien aus.

Ein erfindungsgemäß eingesatzter Vliesstoffüberzug kann antibakteriell ausgerüstet sein, beispielsweise in an sich bekannter Weise durch eine Ausrüstung mit Silber.

Weiterhin kann ein erfindungsgemäße eingesatzter liesstoffüberzug hydrophil, hydrophob oder zonenweise hydrophil und hydrophob im Wechsel ausgerüstet sein.

In einer besonderen Ausgestaltung ist der Vliesstoffüberzug außerdem mit Öffnungen, vorzugsweise Öffnungen mit Durchmessern oder lateralen Abmessungen kleiner 0,3 Millimetern ausgestattet. Diese Öffnungen im Vliesstoff erlauben einen besonders guten Flüssigkeitstransport, beispielsweise beim Abtrocknen von Wunden unter gleichzeitiger Beibehaltung niedriger Reibungskoeifizisnten.

### Kurzbeschreibung der Zeichnung

Nachfolgend wird die Erfindung anhand der beigefügten Zeichnungen näher erläutert. Es zeigen
- Fig. 1: eine elektronenmikroskopische Aufnahme eines Vliesstoffüberzugs in Aufsicht,
- Fig. 2: Fig. 2 eine stärker vergrößerte Aufnahme des in Fig. 1 gezeigten Vliesstoffübetzugs,
- Fig. 3: einen schematischen Querschnitt durch ein Bikomponenten-Filament in Pie-Geometrie und
- Fig.4: eine elektronenmikroskopische Aufnahme der Querschnittsflächen von Kern-Mantel-Flamenten auf Basis von PET / PE.

### Ausführung der Erfindung

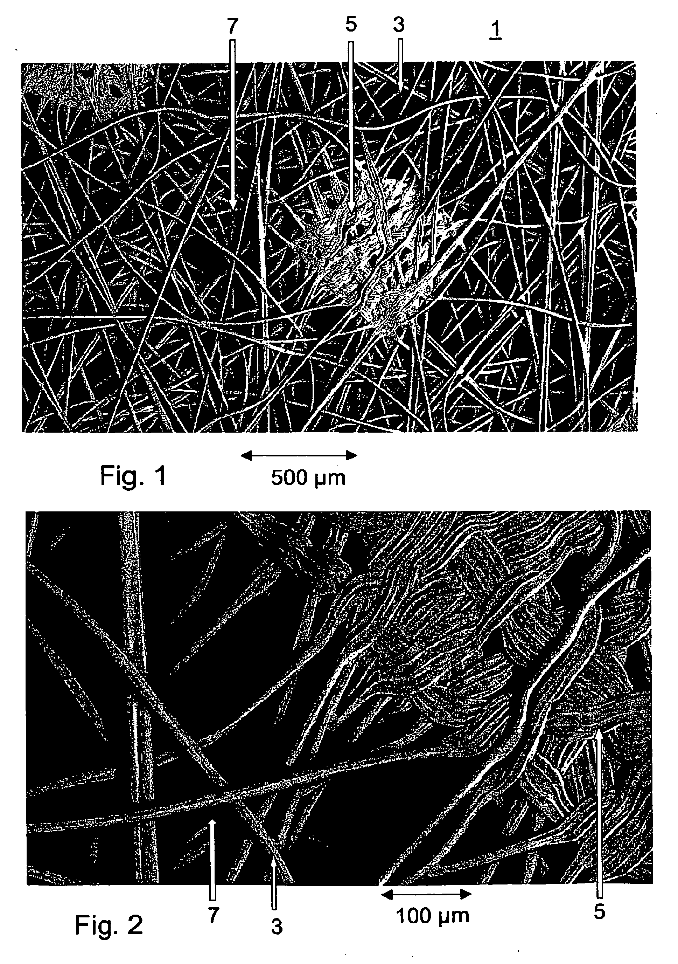

In Fig. 1 ist eine elektronenmikroskopische Aufnahme eines Vliesstoffüberzugs 1 gezeigt. Der Vliesstoffüberzug besteht aus Endlos-Bikomponentenfilamenten 3, die als eine Komponente ein thermoplastisches, heißsiegelbares Material umfassen. Die Filamente 3 sind in ersten, punktförmigen, zueinander beabstandeten Bereichen 5 miteinander verbunden, indem das thermoplastische, heißsiegelbare Material durch lokales Zusammenpressen des Flors und Erwärmen aufgeschmolzen wird. In zweiten Bereichen 7, welche die ersten Bereiche 5 umgeben, sind die Filamente 3 dagegen nicht miteinander verbunden, so dass in diesen Bereichen 7 einerseits eine hohe Durchlässigkeit für Flüssigkeit besteht und andererseits auch eine gewisse Dehnfähigkeit des Vlieses vorhanden ist. Dies gewährleistet, dass dem in den Vliesstoffüberzug eingeschlagenen, beziehungsweise vom Vliesstoffüberzug umgebenen absorbierenden Material eines Tampons oder eines anderen absorbierenden Produkts eine Volumenausdehnungs-Reserve zur Verfügung steht und das absorbierende Material seine flüssigkeitsabsorbierende Eigenschaft unter Volumenausdehnung voll ausschöpfen kann.

Fig. 1 verdeutlicht außerdem, dass die ersten Bereiche 5 nur einen geringen Teil der Oberfläche des Vliesstoffüberzugs 1 einnehmen. Der überwiegende Oberflächenanteil wird demgegenüber von dem oder den Bereichen 7, in welchem die Filamente 3 unverbunden sind, eingenommen.

Bei dem in Fig. 1 gezeigten Beispiel ist insbesondere der Oberflächenanteil der ersten Bereiche 5 kleiner als 25%. Wie anhand des unter der elektronenmikroskopischen Aufnahme dargestellten Maßstabs zu erkennen ist, sind die ersten Bereiche 5 auch relativ klein. Insbesondere sind die lateralen Abmessungen der Bereiche deutlich kleiner als 1 Millimeter. Im Speziellen weisen die Bereiche 5 eine in etwa quadratische Form mit Kantenlängen von ungefähr 500 Mikrometern auf. Die Flächendichte der Bindungspunkte des in Fig. 1 gezeigten Beispiels liegt weiterhin bei über 60 Bindungspunkten pro Quadratzentimeter.

Fig. 2 zeigt in höherer Vergrößerung den Randbereich eines Bindungspunktes. Innerhalb des Bindungspunktes, beziehungsweise des ersten Bereiches 5 ist zu erkennen, dass sich die miteinander verschmolzenen, zusammengepressten Filamente 3 in jeweils mehrere nebeneinander liegende Stränge aufteilen. Diese Stränge werden aus den Sektoren der hochschmelzenden Komponente der Filamente in Bikomponenten-Pie-Geometrie gebildet. Die niedrigschmelzende Komponente ist aufgeschmolzen und wirkt als Schmelzkleber, welcher die Stränge der hochschmelzenden Komponenten der Filamente 3 miteinander verbindet. Bei dem in Fig. 1 und 2 gezeigten Beispiel wurden Pie-Filamente mit 16 Sektoren, also jeweils acht Sektoren der hochschmelzenden und der niedrigschmelzenden Komponente verwendet.

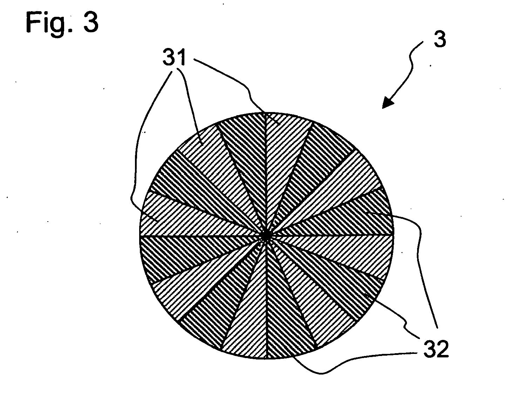

In Fig. 3 ist schematisch ein solches Pie-Filament in Querschnittansicht dargestellt. Das Filament 3 umfasst insgesamt 16 sich jeweils abwechselnde Sektoren 31 und 32. Die Sektoren 31 bestehen dabei aus einem niedrigschmelzenden, heißsiegelbaren Thermoplast, vorzugsweise PE oder PP. Die weiteren Sektoren 32 enthalten höherschmelzendes Polyester-Derivat, vorzugsweise PET. Bei dem in Fig. 3 gezeigten Beispiel sind alle Sektoren gleich groß. Dementsprechend haben die beiden Komponenten auch gleiche Volumenanteile und ähnliche Gewichtsanteile.

Für praktische Anwendungen können die Sektoren 31 und 32 jedoch auch unterschiedliche Winkelabschnitte einnehmen. Insbesondere können die Sektoren 32 aus höherschmelzendem PET auch größer sein, so dass der überwiegende Volumenanteil des Filaments durch diesen Kunststoff gebildet wird. Obwohl PET gegenüber PE einen höheren Reibkoeffizienten aufweist, wird dennoch eine insgesamt niedrige Reibung erzielt. Allgemein wird bevorzugt, wenn die Klebekomponente, beziehungsweise das niedrigschmelzende heißsiegelbare Thermoplast einen Volumenanteil von höchstens 40% aufweist. Besonders bevorzugt wird eine Aufteilung von 70 Gewichtsprozent PET zu 30 Gewichtsprozent PE oder PP.

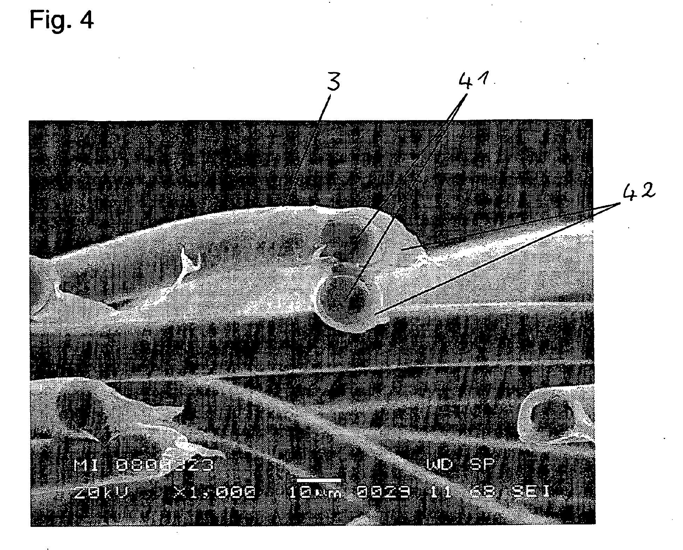

In der elektronenmikroskopischen Aufnahme der Querschnittsflächen von PET / PE-Kem-Mantel-Filamenten von Fig. 4 erkennt man deutlich den Kern 41 der Filamente 3, der von einem Mantel 42 umgeben ist.

in den nachstehenden Tabellen 1 und 2 werden weitere Eigenschaften mehrerer erfindungsgemäßer Vliesstoffüberzüge aufgelistet.

**Tabelle 1: Vliesstoffüberzüge aus Pie-Filamenten auf Basis PE / PET**

| | | Vliesstoff 1 | Vliesstoff 2 | Vliesstoff 3 | Vliesstoff 4 | Vliesstoff 5 | Vliesstoff 6 |
|---|---|---|---|---|---|---|---|
| Polymer | | PE/PET | PE/PET | PE/PET | PE/PET | PE/PET | PE/PET |
| Verhältnis | | 50:50 | 30:70 | 30:70 | 30:70 | 30:70 | 30:70 |
| Querschnittsform | | 16 Pie | 16 Pie | 16 Pie | 16 Pie | 16 Pie | 16 Pie |
| Gewicht | g/m² | 12,0 | 17,0 | 15,0 | 17,0 | 17,3 | 16,3 |
| EN-Dicke | mm | 0,10 | 0,12 | 0,12 | 0,10 | 0,15 | 0,14 |
| HZK Längs | N | 11,0 | 24,2 | 15,5 | 40,6 | 35,1 | 36,3 |
| HZKD Längs | % | 22,0 | 25,8 | 22,0 | 32,0 | 66,9 | 66,7 |
| M 05% Längs | N | 6,0 | 14,5 | 9,5 | 18,5 | 10,2 | 10,9 |
| M 10% Längs | N | 9,0 | 17,5 | 12,0 | 22,5 | 12,3 | 13,4 |
| M 15% Längs | N | 9,5 | 20,0 | 13,5 | 25,5 | 14,4 | 15,6 |
| M 20% Längs | N | 9,5 | 22,0 | 14,5 | 28,1 | 16,4 | 17,7 |
| M 25% Längs | N | 11,5 | 23,0 | 15,0 | 30,8 | 18,5 | 19,9 |
| HZK Quer | N | 7,5 | 24,2 | 18,0 | 32,5 | 20,9 | 21,6 |
| HZK D Quer | % | 24,0 | 33,4 | 30,8 | 32,0 | 57,3 | 54,3 |
| M 05% Quer | N | 4,0 | 11,5 | 8,5 | 11,0 | 6,3 | 5,8 |
| M 10% Quer | N | 5,5 | 14,0 | 10,5 | 13,5 | 8,2 | 8,2 |
| M 15% Quer | N | 6,5 | 16,5 | 12,5 | 15,5 | 9,5 | 9,7 |
| M 20% Quer | N | 7,0 | 19,0 | 14,5 | 17,0 | 10,8 | 11,2 |
| M 25% Quer | N | 7,0 | 21,0 | 16,0 | 18,5 | 12,1 | 12,8 |
| verschweißte Fläche | % | 11,1 | 24,5 | 24,5 | 24,5 | 11,1 | 11,1 |
| Dichte der Verschw. Punkte | P/ cm² | 64,4 | 71,5 | 71,5 | 71,5 | 64,4 | 64,4 |

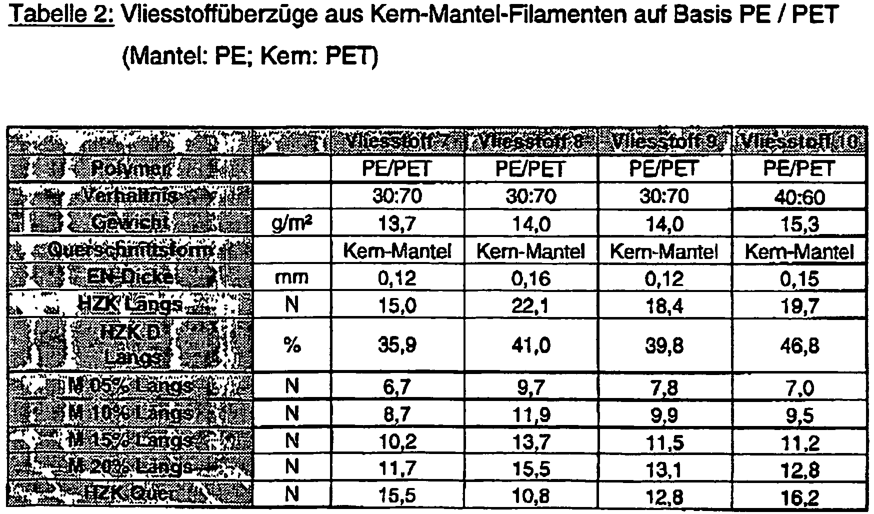

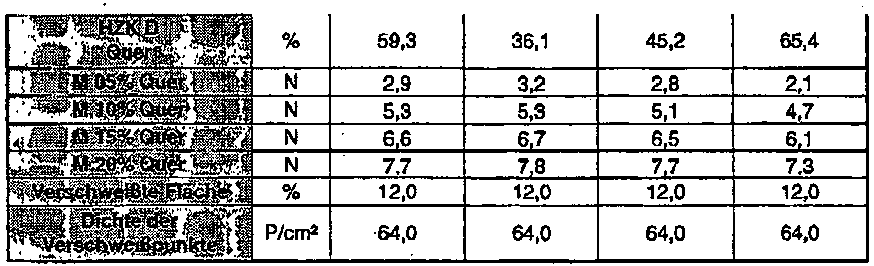

Alle aufgelisteten Vliesstoffüberzüge aus Tabelle 1 wurden aus PE/PET-Bikomponenten-Endlosfilamenten in Pie-Geometrie mit 16 Sektoren hergestellt. Beim Vliesstoff 1 betrug das Verhältnis der beiden Komponenten 50:50. Bei allen anderen gelisteten Vliesen überwiegt die PET Komponente. Die in Tabelle 2 aufgelisteten Vliesstoffüberzüge werden aus PE / PET-Bikomponenten-Endlosfilamenten mit Kem-Mantel-Geometrie mit einem Verhältnis der beiden Komponenten von 30:70 und 40:60 hergestellt.

Bei allen Vliesen liegt der Flächenanteil der ersten Bereiche 5, beziehungsweise der Verschweißungspunkte mit höchstens 24,5 % unter 25%.

Die Größen "M 05%", "M 10%", ... , "M 25%" bezeichnen die Module bei einer Dehnung entsprechend der jeweiligen Prozentangabe. Die Angabe "Längs", beziehungsweise "Quer" bezeichnet die Richtung der Dehnung in Bezug auf die Herstellungsrichtung des Vliesstoffes.

Die Größe "HZK" bezeichnet die Höchstzugkraft. Auch diese Größe ist jeweils für eine Zugbelastung in Längs- und Querrichtung angegeben. Ohne Beschränkung auf die in Tabelle 1 gelisteten Beispiele kann im Falle der Vliesstoffüberzüge aus Pie-Endlosfilamenten eine Höchstzugkraft von im schlechtesten Fall zumindest 7 N erreicht werden. Vliesstoffe, wie die in Tabelle 1 gelisteten Vliesstoffe 2 bis 6, die allgemein mehr als 60 Gewichtsprozent PET, vorzugsweise ein PE/PET-Verhältnis von 30:70 aufweisen, können allgemein auch durch Höchstzugkräfte von zumindest 15 N charakterisiert werden. Im Falle der Vliesstoffüberzüge aus Kem-Mantel-Filamenten in Tabelle 2 wurden bei einem PE/PET-Verhältnis von 30:70 Höchstzugkräfte von mindestens ca. 11 N erreicht.

Die Größe HZK D bezeichnet weiterhin die Dehnung bei Belastung mit der Höchstzugkraft. Wie anhand der Tabellen 1 und 2 ersichtlich ist, weisen alle Beispiele eine Maximaldehnung auf, die unabhängig von der Zugrichtung 22% oder mehr beträgt. Ohne Beschränkung auf die gelisteten Beispiele können erfindungsgemäße Vliesstoffüberzüge daher in Weiterbildung der Erfindung auch zusätzlich durch eine Dehnung bei Höchstzugkraft unabhängig von der Zugrichtung charakterisiert werden, die zumindest 20% beträgt.

Weiterhin wurde die Faserablösung an Tampons mit erfindungsgemäßen Vliesstoffüberzügen aus PIE-Endlos-Bikomponentenfilamenten im Vergleich mit herkömmlichen Tampons in einem Anwendungstest bestimmt. Bei diesem Anwendungstest wurden zunächst die Tampons mittels des Syngina-Standardverfahrens der EDANA in einer synthetischen Vagina getestet. Bei diesem dem Fachmann bekannten Standardverfahren wird eine Testflüssigkeit verwendet, die wie folgt präpariert wird: 10 Gramm Natriumchlorid werden Pro Liter destilliertem oder deionisiertem Wasser werden 10 Gramm Natriumchlorid und 0,5 Gramm Farbstoff zugegeben. Als Farbstoffe kommen folgende Substanzen in Betracht:
- saures Fuchsin, z.B. Fisher F97 Certified Biological Stain, Colour Index N° 42685, Fisher Scientific Company,
- Früchterot-Farbstoff, E 144,
- Cochenillerot, bzw. Ponceau, E 124,
- FD&C Red #40.

In die Kammer der Syngina-Apparatur wird ein Kondom mit einer Reißfestigkeit im Bereich von 17MPa und 30MPa, gemessen entsprechend ASTM D 3492-83 und ASTM D 3492-97 eingesetzt und der Tampon in das Kondom eingeführt. Das Kondom wird in der Kammer mit einem hydrostatischen Druck von 180 ± 10 Millimetern beaufschlagt, während die Testflüssigkeit dem Tampon mittels einer Infusionspumpe über eine Infusionsnadel in einer Menge von 50 ± 2 Millilitem pro Stunde zugeführt wird, wobei die Infusionsnadel das vordere Ende des Tampons berührt.

Der Test wird so lange durchgeführt, bis der erste Tropfen der Testflüssigkeit aus der Vorrichtung austritt, also dementsprechend, bis zu dem Zeitpunkt, ab welchem die Testflüssigkeit durch den Tampon durchsickert.

Nach Beendigung des Syngina-Tests wird der Tampons aus der synthetischen Vagina entfernt und der Aufbau ausgespült. Der Test wurde für jeden Tampontyp mit jeweils drei Tampons durchgeführt. Die ausgespülten Fasern wurden gesammelt, getrocknet und gewogen. Das Ergebnis wurde auf den Faserverlust eines einzelnen Tampons zurückgerechnet.

Die herkömmlichen Tampons wiesen dabei einen Vliesstoffüberzug auf, der ebenso wie die erfindungsgemäßen Vliesstoffe punktweise festgelegt war, allerdings nicht aus Mehrkomponenten-Endlosfilamenten, sondern aus Stapelfasern aufgebaut war. Die Faserablösung betrug bei den herkömmlichen Tampons 2 Milligramm Fasern pro Tampon, während bei den Tampons mit erfindungsgemäßen Vliesstoffüberzügen die Faserablösung abhängig von der Temperatur bei der Verfestigung nur 1 Milligramm betrug oder sogar nicht messbar klein war.

Tampons mit erfindungsgemäßen Vliesstoffüberzügen können daher in Weiterbildung der Erfindung auch so charakterisiert werden, dass deren Faserverlust im Anwendungstest mindestens 50% geringer, vorzugsweise höchstens halb so groß ist, verglichen mit einem Tampon mit einem punktweise verfestigen Stapelfaservlies als Vliesstoffüberzug. Tampons mit erfindungsgernäßern Vliesstoffüberzug sind auch so charakterisiert, dass der Faserverlust eines Tampons im Durchschnitt in einem Syngina-Test höchstens 1,5 Milligramm, vorzugsweise nicht mehr als 1,1 Milligramm, oder sogar kleiner 0,5 Milligramm beträgt.

Die Eigenschaft des allenfalls geringen Faserverlustes und der geringen bis fehlenden Faserenden, wie sie die erfindungsgemäßen Vliessioffüberzüge aufweisen, ist unter anderem deshalb besonders von Vorteil, da damit kleinere Verletzungen und Reizungen des anliegenden Gewebes beim Gebrauch vermindert werden können.

Es ist dem Fachmann ersichtlich, dass die Erfindung nicht auf die vorstehend beschriebenen Ausführungsbeispiele beschränkt ist, sondern vielmehr in vielfältiger Weise variiert werden kann. Insbesondere können die Merkmale der einzelnen Beispiele auch miteinander kombiniert werden.

## Patentansprüche

1. Tampon mit einem Vliesstoffüberzug, welcher wenigstens eine Lage mit Mehrkomponentenfilamenten aufweist, die wenigstens eine Komponente aus einem thermoplastischen heißsiegelbaren Material enthalten, wobei die Mehrkomponentenfilamente Endlosfilamente sind, und wobei die Endlosfilamente in ersten Bereichen zusammengepresst und durch thermische Verbindung festgelegt sind und in zweiten Bereichen zwischen den ersten durch thermische Verbindung festgelegten Bereichen unverbunden vorliegen, **dadurch gekennzeichnet, dass** der Faserverlust des Tampons im Durchschnitt in einem Syngina-Test höchstens 1,5 Milligramm, vorzugsweise nicht mehr als 1,1 Milligramm beträgt.

2. Tampon gemäß Anspruch 1,
dadurch gekannzeichnet, dass
die Mehrkomponentenfilamente Bikomponentenfilamente mit einer thermoplastischen niedrigschmelzenden und einer hochschmelzenden Komponente umfassen.

3. Tampon nach Anspruch 2, **dadurch gekennzeichnet, dass** die hochschmelzende Komponente ausgewählt ist aus der Gruppe der Polyesterderivate und Mischungen derselben ausgewählt ist.

4. Tampon nach Anspruch 3, **dadurch gekennzeichnet, dass** die Gruppe der Polyesterderivate Polyethylenterephthalat (PET), Polytrimethylenterephthalat (PTT), Polybutylenterephthalat (PBT) und Polymilchsäure (PLA) umfasst.

5. Tampon nach einem der Ansprüche 2 bis 4, **dadurch gekennzeichnet, dass** die niedrigschmelzende Komponente ausgewählt ist aus der Gruppe der Polyolefine.

6. Tampon nach Anspruch 5, **dadurch gekennzeichnet, dass** die niedrigschmelzende Komponente Polyethylen und/oder Polypropylen umfasst.

7. Tampon nach einem der vorstehenden Ansprüche, **dadurch gekennzeichnet, dass** zumindest zwei der Komponenten Bereiche der Filamentoberflächen bilden.

8. Tampon nach einem der Ansprüche 2 bis 7, **dadurch gekennzeichnet, dass** die Bikomponentenfilamente eine Pie- oder Kern-Mantel-Geometrie aufweisen.

9. Tampon nach Anspruch 8, **dadurch gekennzeichnet, dass** die Bikomponentenfilamente Pie- oder Kem-Mantel-Filamente aus PET/PE umfassen.

10. Tampon nach Anspruch 9, **dadurch gekennzeichnet, dass** der Anteil an PET in den Pie- oder Kem-Mantel-Filamenten zwischen 10 und 90 Gewichtsprozent, vorzugsweise zwischen 60 und 90 Gewichtsprozent, beträgt.

11. Tampon nach einem der Ansprüche 1 bis 10, **dadurch gekennzeichnet, dass** die wenigstens eine Lage aus Mehrkomponentenfilamenten mittels über die Oberfläche verteilter, voneinander beabstandeter Bindungspunkte thermisch verfestigt und/oder festgelegt ist.

12. Tampon nach Anspruch 11, **dadurch gekennzeichnet, dass** die Flächendichte der Bindungspunkte größer als 60 Punkte pro Quadratzentimeter ist.

13. Tampon gemäß einem der vorstehenden Ansprüche, **dadurch gekennzeichnet, dass** die ersten Bereiche laterale Abmessungen kleiner als 1 Millimeter aufweisen.

14. Tampon gemäß einem der vorstehenden Ansprüche, **dadurch gekennzeichnet, dass** die Fläche der ersten Bereiche einen Anteil an der Gesamtoberfläche hat, der 30%, vorzugsweise 25% nicht überschreitet.

15. Tampon gemäß einem der vorstehenden Ansprüche, **dadurch gekennzeichnet, dass** der Vliesstoffüberzug eine Höchstzugkraft von zumindest 7 N, vorzugsweise zumindest 15 N aufweist.

16. Tampon gemäß einem der vorstehenden Ansprüche, **dadurch gekennzeichnet, dass** der Vliesstoffüberzug eine Dehnung bei Höchstzugkraft unabhängig von der Zugrichtung aufweist, die zumindest 20% beträgt.

17. Tampon gemäß einem der vorstehenden Ansprüche, **dadurch gekennzeichnet, dass** der Vliesstoffüberzug Öffnungen aufweist, vorzugsweise Öffnungen mit Durchmessern oder lateralen Abmessungen kleiner 0,3 Millimetern.

## Claims

1. Tampon with a non-woven covering which includes at least one layer with multi-component filaments which contain at least one component composed of a thermoplastic heat-sealable material, wherein the multi-component filaments are endless filaments, and wherein the endless filaments are in a compressed and thermobondedly defined state in first regions and are in an unbound state in second regions between the first regions defined by thermobonding, **characterized in that** the tampon loses on average not more than 1.5 milligrams and preferably not more than 1.1 milligrams of fibre in a Syngina test.

2. Tampon according to Claim 1, **characterized in that** the multi-component filaments comprise bi-component filaments with a thermoplastic low-melting component and a high-melting component.

3. Tampon according to Claim 2, **characterized in that** the high-melting component is selected from the group of polyester derivatives and mixtures thereof.

4. Tampon according to Claim 3, **characterized in that** the group of polyester derivatives comprise polyethylene terephthalate (PET), polytrimethylene terephthalate (PTT), polybutylene terephthalate (PBT) and polylactic acid (PLA).

5. Tampon according to any of Claims 2 to 4, **characterized in that** the low-melting component is selected from the group of polyolefins.

6. Tampon according to Claim 5, **characterized in that** the low-melting component comprises polyethylene and/or polypropylene.

7. Tampon according to any preceding claim, **characterized in that** at least two of the components form regions of the filament surfaces.

8. Tampon according to any of Claims 2 to 7, **characterized in that** the bi-component filaments have a pie or core-sheath geometry.

9. Tampon according to Claim 8, **characterized in that** the bi-component filaments comprise pie or core-sheath filaments composed of PET/PE.

10. Tampon according to Claim 9, **characterized in that** the proportion of PET in the pie or core-sheath filaments is between 10 and 90 weight per cent, preferably between 60 and 90 weight per cent.

11. Tampon according to any of Claims 1 to 10, **characterized in that** the at least one layer of multi-component filaments is thermally consolidated and/or defined via spaced-apart bonding points distributed over the surface.

12. Tampon according to Claim 11, **characterized in that** the areal density of the bonding points is greater than 60 points per square centimetre.

13. Tampon according to any preceding claim, **characterized in that** the first regions have lateral dimensions smaller than 1 millimetre.

14. Tampon according to any preceding claim, **characterized in that** the area of the first regions comprises a proportion of the total surface area that does not exceed 30%, preferably 25%.

15. Tampon according to any preceding claim, **characterized in that** the non-woven covering has an ultimate tensile strength force of at least 7 N, preferably at least 15 N.

16. Tampon according to any preceding claim, **characterized in that** the non-woven covering has an elongation of at least 20% when subjected to the ultimate tensile strength force irrespective of the tensile direction.

17. Tampon according to any preceding claim, **characterized in that** the non-woven covering has openings, preferably openings with diameters or lateral dimensions below 0.3 millimetres.

## Revendications

1. Tampon présentant un recouvrement en non-tissé, qui présente au moins une couche avec des filaments à plusieurs composants, qui contiennent au moins un composant en un matériau thermoplastique thermoscellable, les filaments à plusieurs composants étant des filaments continus et les filaments continus étant compressés dans des premières zones et fixés par assemblage thermique et se trouvant, dans des deuxièmes zones, entre les premières zones fixées par assemblage thermique, de manière non assemblée, **caractérisé en ce que** la perte des fibres du tampon représente en moyenne, dans un test Syngina, au plus 1,5 milligramme, de préférence pas plus de 1,1 milligramme.

2. Tampon selon la revendication 1, **caractérisé en ce que** les filaments à plusieurs composants comprennent des filaments à deux composants et un composant thermoplastique à bas point de fusion et un composant à point de fusion élevé.

3. Tampon selon la revendication 2, **caractérisé en ce que** le composant à point de fusion élevé est choisi dans le groupe des dérivés de polyester et leurs mélanges.

4. Tampon selon la revendication 3, **caractérisé en ce que** le groupe des dérivés de polyester comprend le poly(téréphtalate d'éthylène) (PET), le poly(téréphtalate de triméthylène) (PTT), le poly(téréphtalate de butylène) (PBT) et le poly(acide lactique) (PLA).

5. Tampon selon l'une quelconque des revendications 2 à 4, **caractérisé en ce que** le composant à bas point de fusion est choisi dans le groupe des polyoléfines.

6. Tampon selon la revendication 5, **caractérisé en ce que** le composant à bas point de fusion comprend du polyéthylène et/ou du polypropylène.

7. Tampon selon l'une quelconque des revendications précédentes, **caractérisé en ce qu'**au moins deux des composants forment des zones des surfaces des filaments.

8. Tampon selon l'une quelconque des revendications 2 à 7, **caractérisé en ce que** les filaments à deux composants présentent une géométrie de type tarte ou noyau-enveloppe.

9. Tampon selon la revendication 8, **caractérisé en ce que** les composants à deux filaments présentent des filaments à géométrie de type tarte ou noyau-enveloppe en PET/PE.

10. Tampon selon la revendication 9, **caractérisé en ce que** la proportion de PET dans les filaments à géométrie de type tarte ou noyau-enveloppe représente entre 10 et 90% en poids, de préférence entre 60 et 90% en poids.

11. Tampon selon l'une quelconque des revendications 1 à 10, **caractérisé en ce que** ladite au moins une couche en filaments à plusieurs composants est consolidée et/ou fixée thermiquement au moyen de points de liaison répartis sur la surface, écartés les uns des autres.

12. Tampon selon la revendication 11, **caractérisé en ce que** la densité de surface des points de liaison est supérieure à 60 points par centimètre carré.

13. Tampon selon l'une quelconque des revendications précédentes, **caractérisé en ce que** les premières zones présentent des dimensions latérales inférieures à 1 millimètre.

14. Tampon selon l'une quelconque des revendications précédentes, **caractérisé en ce que** la surface des premières zones présente une proportion par rapport à la surface totale qui ne dépasse pas 30%, de préférence pas 25%.

15. Tampon selon l'une quelconque des revendications précédentes, **caractérisé en ce que** le recouvrement en non-tissé présente une résistance maximale à la traction d'au moins 7 N, de préférence d'au moins 15 N.

16. Tampon selon l'une quelconque des revendications précédentes, **caractérisé en ce que** le recouvrement en non-tissé présente un allongement à la résistance maximale à la traction, indépendamment du sens de traction, d'au moins 20%.

17. Tampon selon l'une quelconque des revendications précédentes, **caractérisé en ce que** le recouvrement en non-tissé présente des ouvertures, de préférence des ouvertures présentant des diamètres ou des dimensions latérales inférieur(e)s à 0,3 millimètre.
